# EUROPEAN PATENT APPLICATION

(11) **EP 1 464 339 A1**
(43) Date of publication of application: **06.10.2004**
(21) Application number: 02783789.7
(22) Date of filing: 05.12.2002
(51) Int. Cl.: A61K 45/00, A61K 38/22, A61P 9/00, A61P 9/10, A61P 13/12, A61P 43/00

(54) **CELL MIGRATION INHIBITOR**

(30) Priority: 07.12.2001 JP 2001374826
(71) Applicant: Kaken Pharmaceutical Co., Ltd., Bunkyo-ku, Tokyo 113-8650 (JP)
(72) Inventor: AMAKAWA, M. Cent. Res. Lab. Kaken Pharm. Co., Ltd., Kyoto, Kyoto 607-8042 (JP); MURATA, T. Cent. Res. Lab. Kaken Pharm. Co., Ltd., Kyoto, Kyoto 607-8042 (JP)
(74) Representative: HOFFMANN EITLE
(86) International application number: PCT/JP2002/012783
(87) International publication number: WO 2003/047624

(57) **Abstract**

An agent for prevention or treatment of vascular disease and renal dysfunction that are accompanied by the migration of vascular smooth muscle cells and mesangial cells comprises as an active ingredient a growth-hormone secretion promoting substance receptor agonist that have a strong inhibitory action on the migration of vascular smooth muscle cells and mesangial cells. Using such an agent for prevention or treatment, one can suppress arteriosclerosis, recurrence of intimal thickening after angioplasty, and even glomerulosclerosis.

## Description

### TECHNICAL FIELD

This invention relates to a cell migration inhibitor comprising a growth-hormone secretion promoting substance receptor agonist as an active ingredient, in particular, an inhibitor for cell migration as induced by PDGF and angiotensin II, as well as an agent for prevention or treatment of diseases that are accompanied by the migration of vascular smooth muscle cells or mesangial cells.

### BACKGROUND ART

In embryogenesis, cancer, inflammation, etc., cell migration is a phenomenon that precedes the proliferation of the cell. Migration of vascular smooth muscle cells or mesangial cells also occurs before their proliferation and is involved with well-known pathologic states, arteriosclerosis and glomerulonephritis.

In normal vascular smooth muscle cells, the greater part of the cytoplasm is occupied by myofilaments and such cells, being responsible for vascular constriction and relaxation, are classified as a contractile (differentiated) type. In smooth muscle cells observed in focal arteriosclerosis, the content of myofilaments is decreased markedly whereas organelles such as endoplasmic reticula and mitochondria develop to provide the cells with enhanced ability to proliferate and migrate. Smooth muscle cells of these traits are classified as a synthetic (proliferative) type. Transformation of smooth muscle cells to the synthetic type and subsequent abnormal proliferation is hypothesized as a major cause of the progression of arteriosclerosis (Circ. Res. (1986) 58, 427-444). In general, vascular smooth muscle cells, when isolated from a living body and cultured, will rapidly lose the traits of the contractile type and change to the synthetic type.

Regarding the onset and development of arteriosclerosis, as well as stenosis and occlusion of blood vessels, the "response-to-injury hypothesis" proposed by Ross has gained wide acceptance (Nature (1993) 362:801-809). According to this theory, a risk factor such as hypertension or hyperlipidemia damages the endothelium and the damaged endothelial cells or inflammatory cells accumulating at the damaged site release growth factors such as platelet-derived growth factor (hereunder referred to as PDGF) and other vasoactive substances including cytokines, causing the medial smooth muscle cells to migrate to the intima. The migrating smooth muscle cells enhance the proliferative ability and the extracellular matrix-producing activity.

Regimens for preventive drug therapy against arteriosclerosis include administering therapeutics for hypertension and hyperlipidemia in order to reduce the risk factor and administering anti-platelet agents in order to prevent thrombus formation in arteriosclerotic lesions. In current attempts, drugs that inhibit either one of the abilities of vascular smooth muscle cells to migrate, proliferate and produce the extracellular matrix are applied to prevent and treat arteriosclerosis and intimal rethickening after angioplasty. For example, tranilast, although weak with its effective concentration being in the range of 1 x 10⁻⁵ M to 1 x 10⁻³ M, has in vitro capabilities of inhibiting the migration, proliferation and extracellular matrix production of vascular smooth muscle cells (Atherosclerosis (1994) 107:179-185) and is being studied in clinical settings as an agent to prevent restenosis after percutaneous transluminal coronary angioplasty (hereunder referred to as PTCA). In addition, C-type sodium peptide, an endogenous vasoactive peptide has been reported to show a high inhibitory action on the migration of vascular smooth muscle cells in vitro and the peptide itself and the inhibitor of the degradation enzyme hold promise as therapeutics for arteriosclerosis, etc. (Arterioscler. Thromb. Vasc. Biol. (1996) 16:1080-1087).

Mesangial cells originate from arterial smooth muscle cells and their migration and proliferation can potentially progress to renal dysfunction. A glomerulus is a network of capillaries in the form of a tuft and consists of glomerular endothelial cells, mesangial cells that hold capillaries together, glomerular epithelial cells, glomerular basal membrane and mesangial matrix. The glomerular construct is broken by an increase in the mesangial matrix secreted from mesangial cells and this leads to glomerulosclerosis.

The etiology of glomerulonephritis and the mechanism of its progression have not been fully unraveled. One of the theories supported today is the hypothesis proposed by Floege et al. (Kidney Int. (1993) 39:S47-S54). According to this theory, platelets and macrophages are activated in glomeruli to release PDGF and basic fibroblast growth factor, which induce mesangial cell proliferation and resultant glomerulosclerosis. Later studies have confirmed that the proliferation of mesangial cells is preceded by their migration.

The current methods for treating renal dysfunctions such as glomerulonephritis are by no means radical and mostly focus on maintaining renal functions, reducing urinary protein and controlling hypertension and other complications. Renal dysfunctions have a very disappointing prognosis and lead to renal failure in a comparatively short period; a majority of cases require dialysis therapy and eventually result in death. Hence, the advent of a drug capable of drastic treatment is desired.

Growth-hormone secretion promoting substances secrete growth hormone from the pituitary gland via growth-hormone secretion promoting substance receptors present in the hypothalamus and pituitary. Ghrelin is an endogenous ligand for growth-hormone secretion promoting substance receptors, discovered from the stomach extract and is found to be in plasma at high concentrations. It has been verified that growth-hormone secretion promoting substance receptors are also expressed in blood vessels, heart, lung, pancreas, kidney, liver and fats at the protein level or mRNA level; growth-hormone secretion promoting substance in peripheral organs are being actively studied from physiological and pharmacological aspects (Nature (1999) 402:656-660). It has recently been reported that the expression of growth-hormone secretion promoting substance receptors is increased at sites of human coronary arteriosclerotic lesions but the pathophysiological importance of the phenomenon remains to be determined (Brit. J. Pharmacol. (2001) 134:143-149). Rossoni et al. reported that hexarelin, a growth-hormone releasing peptide, improved the hypercontractile responsiveness to endothelin and hyporesponsiveness to acetylcholine in the blood vascular vessels of hypophysectomized rats (J. Cardiovasc. Pharmacol. (1999) 34:454-460). It is suggested that those actions of hexarelin are on vascular endothelial cells. Ghrelin is produced in large amounts in the kidney (FEBS Lett. (2000) 486:213-216) but no report has been made of the role (if any) that may be played by ghrelin in the kidney.

### DISCLOSURE OF THE INVENTION

An object of the present invention is to discover compounds that have a strong inhibitory action on the migration of vascular smooth muscle cells and mesangial cells, thus being able to suppress arteriosclerosis, the intimal rethickening after angioplasty, and even glomerulosclerosis.

The present inventors conducted intensive studies with a view to attaining the stated object. Surprisingly, they found that growth-hormone secretion promoting substance receptor agonists have a strong inhibitory action on the migration of vascular smooth muscle cells and mesangial cells. The present invention has been accomplished on the basis of this finding.

### BRIEF DESCRIPTION OF THE DRAWINGS

Fig. 1 is a graph showing the suppressive action of KP-102 on the PDGF-induced migration of human vascular smooth muscle cells;
Fig. 2 is a graph showing the suppressive action of ghrelin on the PDGF-induced migration of human vascular smooth muscle cells;
Fig. 3 is a graph showing the suppressive action of KP-102 and ghrelin on the PDGF-induced migration of rabbit vascular smooth muscle cells;
Fig. 4 is a graph showing the suppressive action of GH, GHRH, hexarelin, MK-0677, NN-703, CP-424,391, S-38855 and GHRP-6 on the PDGF-induced migration of human vascular smooth muscle cells;
Fig. 5 is a graph showing the antagonistic action of L-756867 on the migration suppressive action of KP-102, ghrelin and hexarelin on the PDGF-induced migration of human vascular smooth muscle cells;
Fig. 6 is a graph showing the suppressive action of KP-102 on the angiotensin II induced migration of human vascular smooth muscle cells;
Fig. 7 is a graph showing the suppressive action of ghrelin on the angiotensin II induced migration of human vascular smooth muscle cells;
Fig. 8 is a graph showing the suppressive action of KP-102 on the PDGF-induced migration of human mesangial cells; and
Fig. 9 is a graph showing the action of ghrelin on the PDGF-induced migration of human mesangial cells.

In the Figures, * and ** denote significant differences at P values of 5% and 1%, respectively, to the group treated with a solvent containing a migration stimulant; ## denotes a significant difference between two compared groups at a P value of 1%.

### BEST MODES FOR CARRYING OUT THE INVENTION

According to an embodiment of the present invention, there is provided a cell migration inhibitor that comprises a growth-hormone secretion promoting substance receptor agonist as an active ingredient.

The term "cell migration" as used herein means the migration of cells as induced by physiologically active substances such as PDGF and angiotensin II that have migration activity. For example, if an isolated blood vessel is immersed in a PDGF solution, smooth muscle cells come out to migrate. Cell migration inhibitors inhibit such migration and the inhibitory activity is quantitatively measured by the use of a Boyden's chamber as described in the Example below.

According to another aspect of the invention, there is provided an agent for prevention or treatment of disease that is accompanied by the migration of vascular smooth muscle cells or a renal dysfunction that is accompanied by the migration of mesangial cells, characterized in that it comprises a growth-hormone secretion promoting substance receptor agonist as an active ingredient.

According to still another embodiment of the invention, there is provided an agent for prevention or treatment of disease that is accompanied by PDGF- or angiotensin II induced cell migration, characterized in that it comprises a growth-hormone secretion promoting substance receptor agonist as an active ingredient.

The term "disease that is accompanied by the migration of vascular smooth muscle cells" refers to a disease in which for one or other reason, vascular smooth muscle cells migrate from the media to the intima or intimal vascular smooth muscle cells migrate further toward the lumen, where they proliferate or produce the extracellular matrix to cause intimal thickening. Examples of such disease include arteriosclerosis obliterans, coronary arteriosclerosis and restenosis after PTCA. The disease that is accompanied by the migration of vascular smooth muscle cells is not restricted to blood vessels that govern specified organs. Nor is it restricted to the thickening of arterial vessels and it may include occlusive lesions on venous vessels such as shunted vessels of the veins of patients on dialysis.

The term "angioplasty" as used herein refers to surgery employed in the treatment of coronary artery disease associated with arteriosclerotic lesions including angina pectoris and myocardial infarction and it may be exemplified by percutaneous transluminal coronary angioplasty, atherectomy, coronary artery bypassing and stent placement.

The term "renal dysfunction that is accompanied by the migration of mesangial cells" as used herein refers to a condition in which for one or other reason, mesangial cells migrate to a damaged site, where they proliferate or produce the mesangial matrix to pressurize glomeruli and impair their function; examples include renal failure, acute glomerulonephritis, chronic glomerulonephritis, diabetic nephropathy and nephrosclerosis. The disease under consideration is not restricted to primary types but may encompass infectious glomerulopathy and other cases of secondary glomerulopathy associated with multi-organ disease.

The term "growth-hormone secretion promoting substance receptor (hereunder GHSR)" as used herein refers to receptors of growth-hormone releasing peptides and growth-hormone secretagogues and they are typically responsible for the secretion and release of growth hormone (hereunder GH) in the pituitary gland and as for the GHSRs that are expressed in other organs (e.g. heart, lung, pancreas, etc.) including their subtypes, their functions are not restricted to known GH secretion or promotion of GH secretion.

The term "growth-hormone secretion promoting substance receptor agonist (GHSR agent)" as used herein refers to a substance that has the activity to act on GHSR and impart some cell response stimulation. Encompassed by the term are growth-hormone releasing peptides and growth-hormone secretagogues but it is not restricted to those which are represented by GH secretion or promoted GH secretion. The agent is also not restricted to substances that act on GHSR invariably expressed in the organs of all species of animals and includes substances that act on receptors expressed artificially by gene transfer into cell lines.

The term "growth-hormone releasing peptide (hereunder GHRP)" as used herein means peptides having such a pharmacological activity that they act on GHSR to promote GH secretion, wherein endogenous peptides as well as synthetic equivalents and various derivatives thereof are also embraced as long as they have comparable activity.

The term "growth-hormone secretagogue (hereunder GHS)" as used herein refers to non-peptide substances having such a pharmacological activity that they act on GHSR to promote GH secretion, wherein various derivatives thereof are also encompassed as long as they have comparable activity.

Exemplary growth-hormone secretion promoting substance receptor agonists include GHRPs such as pralmorelin, hexarelin and GHRP-6; synthetic GHSs such as ibutamorelin methanesulfonate, tabimorelin and capromorelin; in vivo GHSR ligands such as ghrelin, GHSR ligand polypeptides (WO 01/32705) and adenosine.

Further examples of the growth-hormone secretion promoting substance receptor agonist are disclosed in the following patents, which are provided for illustrative purposes only and should not be taken as limiting.

WO00/10565, WO00/01726, WO99/64456, WO99/58501, WO99/45029, WO99/36431, WO99/09991, WO99/08699, WO98/58950, WO98/58949, WO98/58948, WO98/58947, WO98/51687, WO98/50036, WO98/46569, WO98/46220, WO98/25897, WO98/25622, WO98/16527, WO98/16527, WO98/10653, WO98/03473, WO97/46252, WO97/42223, WO97/40071, WO97/40023, WO97/39768, WO97/34604, WO97/27298, WO97/25057, WO97/24369, WO97/23508, WO97/22622, WO97/22620, WO97/22367, WO97/21730, WO97/18233, WO97/15574, WO97/15573, WO97/15191, WO97/11697, WO97/00894, WO96/38471, WO96/35713, WO96/33189, WO96/32943, WO96/32126, WO96/24587, WO96/24580, WO96/22997, WO96/22782, WO96/15148, WO96/13265, WO96/10040, WO96/05195, WO96/02530, WO95/34311, WO95/17423, WO95/17422, WO95/16707, WO95/16692, WO95/16675, WO95/14666, WO95/13069, WO95/12598, WO95/09633, WO95/03290, WO95/03289, WO94/19367, WO94/18169, WO94/13696, WO94/11397, WO94/11012, WO94/08583, WO94/07519, WO94/07486, WO94/07483, WO94/05634, WO93/04081, WO92/16524, WO92/01711, WO89/10933, WO89/07111, WO89/07110, WO83/02272, USP 6,278,000, USP 6,248,717, USP 6,110,932, USP 5,936,089, USP 5,877,182, USP 5,872,100, USP 5,854,211, USP 5,830,433, USP 5,817,654, USP 5,807,985, USP 5,804,578, USP 5,798,337, USP 5,783,582, USP 5,777,112, USP 5,776,901, USP 5,773,448, USP 5,773,441, USP 5,767,124, USP 5,767,118, USP 5,767,085, USP 5,731,317, USP 5,726,319, USP 5,726,307, USP 5,721,251, USP 5,721,250, USP 5,691,377, USP 5,672,596, USP 5,668,254, USP 5,663,171, USP 5,663,146, USP 5,656,606, USP 5,652,235, USP 5,646,301, USP 5,635,379, USP 5,583,130, USP 5,578,593, USP 5,576,301, USP 5,559,128, USP 5,545,735, USP 5,536,716, USP 5,534,494, USP 5,506,107, USP 5,494,919, USP 5,492,920, USP 5,492,916, USP 5,486,505, USP 5,486,505, USP 5,434,261, USP 5,430,144, USP 5,416,073, USP 5,374,721, USP 5,317,017, USP 5,310,737, USP 5,284,841, USP 5,283,241, USP 5,206,235, USP 5,030,630, USP 4,880,777, USP 4,851,408, USP 4,650,787, USP 4,485,101, USP 4,411,890, USP 4,410,513, USP 4,410,512, USP 4,228,158, USP 4,228,157, USP 4,228,156, USP 4,228,155, USP 4,226,857, USP 4,224,316, USP 4,223,021, USP 4,223,020, USP 4,223,019.

If desired, two or more of the growth-hormone secretion promoting substance receptor agonists or salts thereof may be employed in combination.

The growth-hormone secretion promoting substance receptor agonists for use in the invention can be formulated by known pharmaceutical formulating procedures into various dosage forms such as injection, solution, tablet, powder, subtilized granule, granule and drug delivery system (e.g. slow-releasing agent) either alone or in combination with pharmacologically acceptable carriers and additives. The content of the growth-hormone secretion promoting substance receptor agonists is not limited to any particular value as long as the desired effect is obtained.

Examples of the carriers and additives that can be used to formulate the agents for prevention or treatment of the invention include the following which are commonly employed in preparing of medicines: aqueous solvents such as physiological saline, water (e.g. tap water, distilled water, purified water and water for injection) and Ringer's solution; non-aqueous solvents such as oily solvents (e.g. vegetable oil) and water-soluble solvents (e.g. propylene glycol, macrogol, ethanol and glycerin); bases such as cacao buffer, polyethylene glycol, microcrystalline wax, bleached beeswax, liquid paraffin and white petrolatum; excipients such as sucrose, starch, mannitol, sorbitol, lactose, glucose, cellulose, talc, calcium phosphate and calcium carbonate; binders such as cellulose, methyl cellulose, hydroxypropyl cellulose, poly(propylpyrrolidone), gelatin, gum arabic, polyethylene glycol, sucrose and starch; disintegrants such as starch, carboxymethyl cellulose, hydroxypropyl starch, sodium hydrogencarbonate, calcium phosphate, carboxymethyl cellulose calcium and calcium citrate; lubricants such as magnesium stearate, talc and sodium lauryl sulfate; flavoring agents such as citric acid, menthol, glycine, sorbitol and orange powder; preservatives and antiseptics such as paraoxybenzoate ester, benzyl alcohol, chlorobutanol and quaternary ammonium salts (e.g. benzalkonium chloride and benzethonium chloride); stabilizers such as albumin, gelatin, sorbitol and mannitol; suspending agents such as methyl cellulose, poly(vinylpyrrolidone) and aluminum stearate; plasticizers such as glycerin and sorbitol; dispersants such as hydroxypropylmethyl cellulose; solvent promoters such as hydrochloric acid; emulsifiers such as sodium monostearate; and osmolarity modifiers including electrolytes such as sodium chloride and non-electrolytes such as sugar alcohols, saccharides and alcohols.

The pharmaceutical preparations of the invention are usually administered into mammals including human by various means including intravenous injection, oral application, subcutaneous application, nasal application, transpulmonary application and intra-arterial injection. The dosage of the pharmaceutical preparations varies with the patient's age, body weight, symptoms, the route of administration and other factors; for use on adults, pralmorelin, for example, may be administered in the following manner: ca. 0.1 - 1000 µg per kg of body weight is infused or administered intravenously in one to several portions per day; alternatively, ca. 10 µg - 10 mg per kg of body weight is administered orally in several portions.

The agent for prevention or treatment of the invention can be applied either simultaneously with or a certain time before or after therapeutics for circulatory disease and renal disease, such as coronary dilators, diuretics, inotropic agents, ACE inhibitors, hypertension therapeutics and anti-platelet agents.

The present inventors have found for the first time that growth-hormone secretion promoting substance or growth-hormone secretion promoting substance receptor agonists are effective as drugs that prevent or treat disease that is accompanied by the migration of vascular smooth muscle cells and mesangial cells. The agent for prevention or treatment of the invention which comprises a growth-hormone secretion promoting substance receptor agonist as an active ingredient present high migration inhibitory activity by directly acting on vascular smooth muscle cells or mesangial cells; it also has low toxicity on various animals and feature high safety for humans.

The following example and tests are provided for further illustrating the present invention but are in no way to be taken as limiting.

### [Example 1] Pralmorelin solution

Pralmorelin dihydrochloride (hereunder KP-102) was dissolved in physiological saline (Japanese Pharmacopoeia, or JP) and three solutions having concentrations of 0.1%, 0.2% and 1.0% were prepared.

### [Test 1] Effect of growth-hormone secretion promoting substance receptor agonists on the migration of vascular smooth muscle cells

### 1. Materials

KP-102 (the compound referred to as GHRP-2 in WO 93/04081 was prepared as a dihydrochloride in the usual manner), ibutamorelin methanesulfonate (as prepared by the method described in WO 94/13696: the compound of Example 55, hereunder referred to as MK-0677), tabimorelin (as prepared by the method described in WO 97/23508: the compound of Example 1, hereunder referred to as NN-703), capromorelin (as prepared by the method described in WO 98/58949: the compound of Example 20, hereunder referred to as CP-424,391), S-38855 (as prepared by the method described in WO 00/48623: the compound of Example 4), and hexarelin (PENINSULA LABORATORIES) were each dissolved in dimethyl sulfoxide (Nacalai Tesque, Inc.) and used after being diluted to specified concentrations with water for injection (JP, Otsuka Pharmaceutical Co., Ltd.) containing 1 mg/mL bovine serum albumin (SIGMA). Human-type ghrelin (Peptide Institute, Inc.), human-type GHRH (Peptide Institute, Inc.), recombinant human growth hormone (Wako Pure Chemical Industries, Ltd.), GHRP-6 (BACHEM) and L-756867 (BACHEM) were used after being diluted to specified concentrations with water for injection containing 1 mg/mL bovine serum albumin. The other reagents used were a smooth muscle cell basal medium (SmBM of Sanko Junyaku Co., Ltd.), additive factors (SmGM-2 additive factor set of Sanko Junyaku Co., Ltd. consisting of a human epitheliocyte proliferation factor, a human fibroblast growth factor, insulin, fetal bovine serum, gentamicin and amphotericin B), DMEM (GIBCO), fetal bovine serum (GIBCO), trypsin (GIBCO), PDGF (Genzyme) and angiotensin II (SIGMA).

### 2. Methods

### (1) Cultivation of vascular smooth muscle cells

Human vascular smooth muscle cells were cultured in SmBM with the additive factors in a culture incubator (37°C, 5% CO₂/95% air) and cells at the 5th-9th passage were used in the test. Rabbit vascular smooth muscle cells were obtained by an explant technique from the media of rabbit thoracic aorta and cultured in DMEM with 10% fetal bovine serum; cells at the 5th-7th passage were used in the test.

### (2) Assay of migratory activity

Cells that proliferated to confluence were detached with trypsin, washed twice with an additive factor free medium, dispersed in SmBM containing 0.1% fetal bovine serum and prepared to a cell density of 5 x 10⁵ cells/mL. Migration of cells was assayed according to Koyama et al. method (J. Biol. Chem. (1993) 268:13301-13308) using a 48-well Boyden's chamber (Neuro probe). To be more specific, 28 µL of SmBM containing 10 ng/mL of PDGF and 0.1% FBS was placed in the lower chamber and 50 µL of a cell dispersion preliminarily incubated with each assay compound for 10 minutes was placed in the upper chamber. The upper chamber and the lower chamber were separated by a polycarbonate filter (having pores 8 µm in diameter) precoated with type I collagen (Nitta Gelatin Inc.) Since the placement of the cell dispersion, the Boyden's chamber was incubated in a culture incubator (37°C, 5% CO₂/95% air) for 4 hours. The cells on the upper side of the filter were scraped off and the cells that had migrated to the lower side of the filter were fixed in methanol and stained with Diff-Quick staining solution (International Reagents Corporation). The filter had been air-dried on a glass slide and sealed with Enteran New. The cells were counted under a high-power microscope (x 200) in 4 fields per well and the number of cells that had migrated was averaged to determine the migration activity.

### (3) Statistical analysis

The result of each treated group was represented by the mean ± standard deviation for 3 wells. Statistical significance was estimated by one-way ANOVA, if p value was less than 5%, followed by Dunnett method to compare between vehicle-treated group and the others in the presence of a migration inducer. Values of p less than 5% were considered to be significant.

### 3. Results and discussion

An insult to the vascular endothelium causes platelets to aggregate and release PDGF, etc. PDGF is also secreted by monocyte-derived macrophages invading under the injured endothelium. The PDGF stimulates the medial smooth muscle cells and works as a strong migration factor to promote their proliferation, thus playing an important role at the early phase of arteriosclerosis and the intimal thickening after angioplasty.

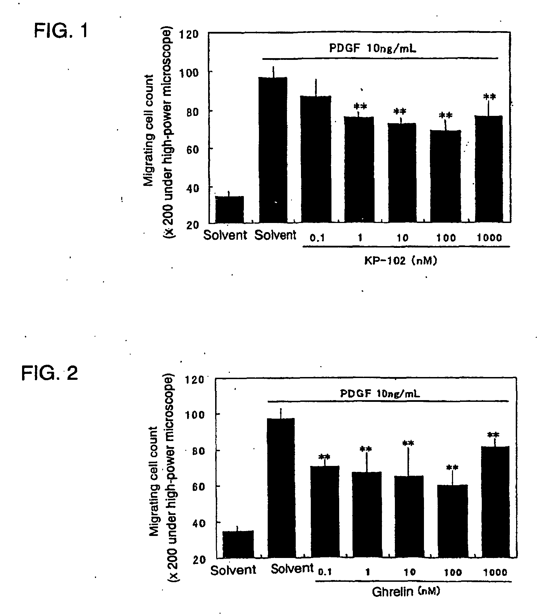

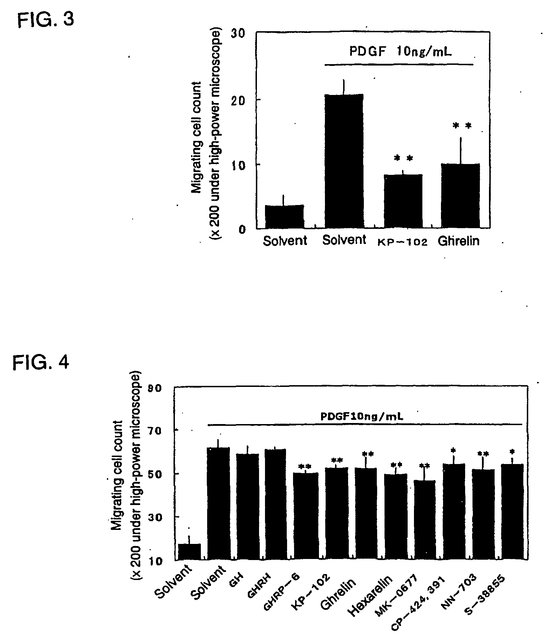

The migration of human vascular smooth muscle cells as induced by 10 ng/mL of PDGF was significantly suppressed by 1 nM - 1000 nM of KP-102, with concentration dependency observed in the range of 1 nM - 100 nM (Fig. 1). Ghrelin showed a suppressive action between 0.1 nM and 1000 nM, which was concentration dependent in the range of 0.1 nM - 100 nM (Fig. 2). We also carried out assays using rabbit vascular smooth muscle cells and found that both KP-102 and ghrelin showed a significant suppressive action in the treatment with 10 nM (Fig. 3). Furthermore, we examined the efficacy of hexarelin, MK-0677, NN-703, CP-424,391, S-38855 and GHRP-6 which were known growth-hormone secretion promoting substance receptor agonists on the migration of human vascular smooth muscle cells; each compound showed a significant suppressive action at a concentration of 10 nM. However, no action was exhibited by growth hormone (GH) at 0.1 µg/mL or growth-hormone releasing hormone (GHRH) at 10 nM (Fig. 4).

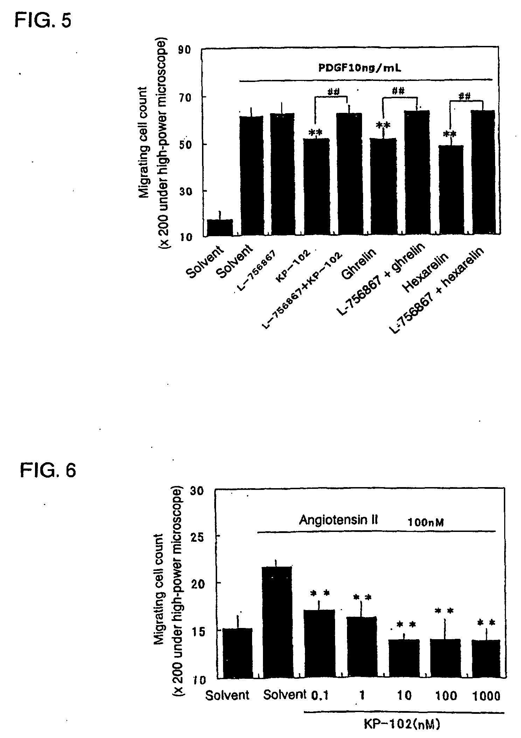

Next, we determined how the GHS receptor antagonist L-756867 affect the suppressive action of KP-102, ghrelin and hexarelin in the migration of vascular smooth muscle cells; 1000 nM of L-756867 was found antagonistic to the migration suppressing action of KP-102, ghrelin or hexarelin at 10 nM (Fig. 5).

As shown above, the various growth-hormone secretion promoting substance receptor agonists with different structures suppressed the PDGF-induced cell migration and lost their action upon treatment with the GHS receptor antagonist; it is therefore clear that the growth-hormone secretion promoting substance receptor agonists do not suppress cell migration as antagonists of the PDGF receptor but suppress cell migration by a GHSR-mediated mechanism.

The new mechanism of cell migration we found suggested to us the possibility of suppressing migration induced by other factors. It is well known that the renin-angiotensin system plays an important role in the circulation regulatory system. Angiotensin II usually works for vasoconstriction but works for stimulation of migration of vascular smooth muscle cells if they are transformed to the synthetic type.

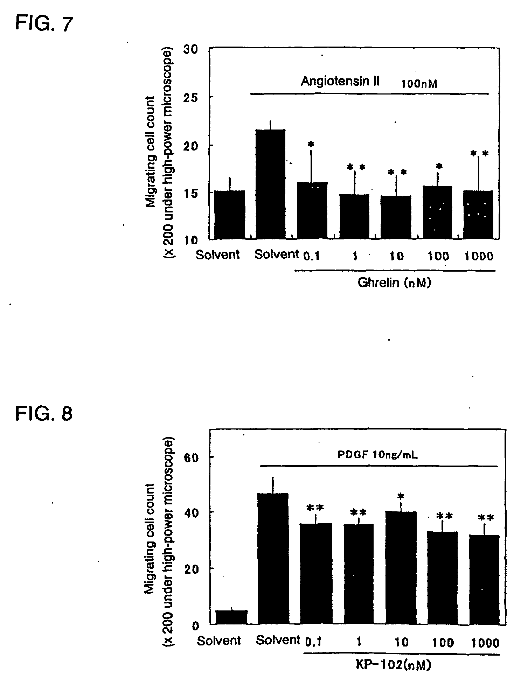

The migration of human vascular smooth muscle cells as induced by 100 nM of angiotensin II was significantly suppressed by 0.1 nM - 1000 nM of KP-102 (Fig. 6) and it was also suppressed significantly by 0.1 nM - 1000 nM of ghrelin (Fig. 7). Thus, it became clear that the growth-hormone secretion promoting substance receptor agonists can suppress not only the migration induced by PDGF but also the migration induced by angiotensin II.

Therefore, the agent for prevention or treatment of the invention which comprises the growth-hormone secretion promoting substance receptor agonist as an active ingredient suppresses the migration of vascular smooth muscle cells and works effectively as an agent for preventing or treating arteriosclerosis and disease that is accompanied by the intimal thickening after angioplasty.

It should be added that the growth-hormone secretion promoting substance receptor agonists inhibited not only the migration of human-derived cells but also the migration of rabbit-derived cells. Hence, the migration inhibitory action of interest is not restricted to humans. The growth-hormone secretion promoting substance receptor agonists are also effective as an agent for preventing or treating arteriosclerosis and other life-style related disease in companion animals.

### [Test 2] Effect of growth-hormone secretion promoting substance receptor agonists on the migration of mesangial cells

### 1. Materials and methods

Human mesangial cells (BioWhittaker) were cultivated in MsGM [basal medium for mesangial cells (Sanko Junyaku Co., Ltd.) containing an MsGM additive factor set (Sanko Junyaku Co., Ltd.; consisting of FBS, gentamicin and amphotericin B)] and cells at the 5th-7th passage were used in the test. The other materials and methods were the same as in Test 1.

### 2. Results and discussion

Mesangial cells integrate endothelial cells in a renal glomerulus and regulate the glomerular blood flow by contracting and relaxing. An injury or dysfunction of the endothelium activates platelets and the aggregated platelets, as well as the monocyte-derived macrophages invading under the endothelium will secrete PDGF. The PDGF works as a strong migration factor for mesangial cells and promotes not only their proliferation but also the production of the mesangial matrix. These actions of PDGF are beneficial to the remodeling of tissues; on the other hand, excessive actions of PDGF may cause glomerulosclerosis.

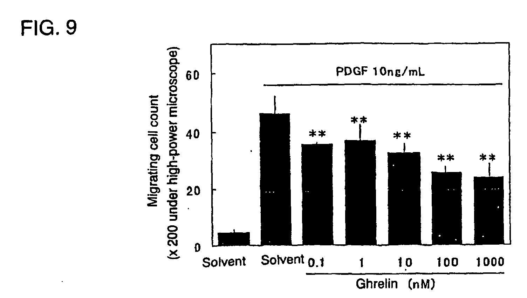

GHSR has been verified to upregulate in the kidney but its functions are yet to be known. The present inventors therefore studied the action of growth-hormone secretion promoting substance receptor agonists on the migration of mesangial cells. PDGF-induced migration was significantly suppressed by KP-102 at concentrations of 0.1 nM and more (Fig. 8). This action was also verified in the use of ghrelin (Fig. 9).

Migration is a characteristic phenomenon recognized on mesangial cells. As shown in Test 1, the suppression of migration by growth-hormone secretion promoting substance receptor agonists is not an antagonistic action in PDGF receptors but it is a GHSR-mediated event which would also easily suppress the migration due to other migration factors. Therefore, the growth-hormone secretion promoting substance receptor agonists would be effective in the treatment of various renal diseases triggered by the migration of mesangial cells.

### INDUSTRIAL APPLICABILITY

In Test 1, KP-102, hexarelin, GHRP-6, ghrelin, MK-0677, NN-703, CP-424, 391 and S-38855 showed potent inhibitory action on the PDGF-induced migration of vascular smooth muscle cells and they are in the classes of GHRP and GHS; at the same time, KP-102, hexarelin, GHRP-6, MK-0677, NN-703 (Eur. J. Endocrinol. (1999) 141:180-189), ghrelin (Nature (1999) 402:656-660) and CP-424,391 (Endocrine (2001) 14:121-132) are growth-hormone secretion promoting substance receptor agonists. Speaking of L-756867, it is known as a GHS receptor antagonist that binds to growth-hormone secretion promoting substance receptors (Mol. Endocrinol. (1996) 10:57-61) and suppresses the GHRP- or GHS-stimulated GH secretion from pituitary gland cells (J. Endocrinol. (1997) 152:155-158). In Test 1, the migration suppressing action of KP-102, ghrelin and hexarelin was antagonized by L-756867. The growth hormone (GH) and the growth-hormone releasing hormone (GHRH) showed no action on the PDGF-induced migration of vascular smooth muscle cells. These facts show that the cell migration suppressing action of the growth-hormone secretion promoting substance receptor agonists is a GHSR-mediated action.

Further, the results of Tests 1 and 2 easily lead us to conjecture that the suppressive action of the growth-hormone secretion promoting substance receptor agonists on cell migration is not necessarily limited to vascular smooth muscle cells and mesangial cells but is also effective against the migration of other cells, say, myofibroblasts since vascular smooth muscle cells and mesangial cells become myofibroblast-like through transformation. It should be noted that myofibroblasts also migrate upon stimulation with PDGF. It has been pointed out that myofibroblasts are associated with fibrogenesis in organs as exemplified by cirrhosis, tubulointerstitial disorder and pulmonary fibrosis and thus GHS would also be effective as an agent for preventing or treating such diseases.

## Claims

1. A cell migration inhibitor that comprises a growth-hormone secretion promoting substance receptor agonist as an active ingredient.

2. A PDGF-induced cell migration inhibitor that comprises a growth-hormone secretion promoting substance receptor agonist as an active ingredient.

3. An angiotensin II-induced cell migration inhibitor that comprises a growth-hormone secretion promoting substance receptor agonist as an active ingredient.

4. A vascular smooth muscle cell migration inhibitor that comprises a growth-hormone secretion promoting substance receptor agonist as an active ingredient.

5. An agent for prevention or treatment of a vascular disease that is accompanied by the migration of vascular smooth muscle cells, said agent comprising a growth-hormone secretion promoting substance receptor agonist as an active ingredient.

6. The agent for prevention or treatment according to claim 5, wherein said vascular disease is arteriosclerosis.

7. The agent for prevention or treatment according to claim 5, wherein said vascular disease is one accompanied by the rethickening of the vascular intima after angioplasty.

8. A mesangial cell migration inhibitor comprising a growth-hormone secretion promoting substance receptor agonist.

9. An agent for prevention or treatment of renal dysfunction accompanied by the migration of mesangial cells, which comprises a growth-hormone secretion promoting substance receptor agonist.

10. The agent for prevention or treatment according to claim 9, wherein said renal dysfunction is glomerulonephritis.

11. The agent for prevention or treatment according to claim 10, wherein said glomerulonephritis is mesangial proliferative glomerulonephritis.

12. A myofibroblast migration inhibitor that comprises a growth-hormone secretion promoting substance receptor agonist as an active ingredient.

13. An agent for prevention or treatment of fibrogenesis in organs that comprises a growth-hormone secretion promoting substance receptor agonist as an active ingredient.

14. The cell migration inhibitor according to any one of claims 1, 2, 3, 4, 8 and 12, wherein the growth-hormone secretion promoting substance receptor agonist is a growth-hormone secretagogue and/or a growth-hormone releasing peptide.

15. The agent for prevention or treatment according to any one of claims 5, 6, 7, 9, 10, 11 and 13, wherein the growth-hormone secretion promoting substance receptor agonist is a growth-hormone secretagogue and/or a growth-hormone releasing peptide.
